# EUROPÄISCHE PATENTSCHRIFT

(11) **EP 1 294 426 B1**
(45) Veröffentlichungstag und Bekanntmachung des Hinweises auf die Patenterteilung: **27.06.2007**
(21) Anmeldenummer: 01962819.7
(22) Anmeldetag: 02.07.2001
(51) Int. Cl.: A61M 16/00

(54) **Vorrichtung zur Erfassung der Atmungstätigkeit einer Person**
Device for detecting the respiratory activity of a person
Dispsotif de détection de l'activité respiratoire d'une personne

(30) Priorität: 30.06.2000 DE 10031079
(43) Veröffentlichungstag der Anmeldung: 26.03.2003
(73) Patentinhaber: MAP Medizin-Technologie GmbH, 82152 Martinsried (DE)
(72) Erfinder: MADAUS, Stefan, 82152 Krailling (DE); SCHNEIDER, Hartmut, Lutherville, MD 21093 (US); JAKOBS, Rainer, 81539 München (DE); VÖGELE, Harald, 82131 Gauting (DE)
(74) Vertreter: Heunemann, Dieter
(86) Internationale Anmeldenummer: PCT/EP2001/007574
(87) Internationale Veröffentlichungsnummer: WO 2002/000283

(56) Entgegenhaltungen:
- WO-A-93/09834
- WO-A-94/23780
- WO-A-95/32016

## Beschreibung

Die Erfindung betrifft eine Vorrichtung zur Erfassung der Atmungstätigkeit einer Person sowie zur Steuerung des zeitlichen Verlaufes des Druckes von Atemgas gemäß dem Oberbegriff von Patentanspruch 1.

Auf Grundlage einer CPAP-Therapie ist es möglich, schlafbezogenen Atmungsstörungen auf physiologisch gut verträgliche Weise vorzubeugen.

Durch die Zufuhr des Atemgases unter einem gegenüber dem Umgebungsdruck definiert angehobenen Druckniveau wird es möglich, eine pneumatische Schienung der oberen Atemwege zu erreichen, wodurch auf wirkungsvolle Weise etwaigen Obstruktionen in diesem Bereich vorgebeugt werden kann oder zumindest im Rahmen einer vorübergehenden Verengung der Atemwege eine ausreichende Sauerstoffversorgung des Patienten sichergestellt ist. Im Hinblick auf eine möglichst hohe physiologische Verträglichkeit wird allgemein ein möglichst niedriges Beatmungsdruckniveau, bei welchem eine ausreichende pneumatische Schienung der oberen Atemwege gewährleistet ist, angestrebt. Es hat sich gezeigt, daß dieser Druckpegel bei bestimmten Patienten erheblichen Schwankungen unterliegt. Um diesen Schwankungen des erforderlichen Therapiedruckes Rechnung zu tragen, sind Versuche mit sogenannten Auto-CPAP-Geräten unternommen worden, die beispielsweise beim Auftreten von Schnarchgeräuschen selbsttätig eine vorübergehende Erhöhung des Therapiedrucks vornehmen. Es sind auch CPAP-Geräte bekannt, bei welchen der zeitliche Verlauf des Atemgasstromes erfaßt wird und auf etwaige Anzeichen für eine Atemwegsobstruktion analysiert wird. Falls Anzeichen für eine Atemwegsobstruktion vorliegen, wird ebenfalls eine vorübergehende Anhebung des Therapiedruckes veranlaßt.

Es sind auch Auto-CPAP-Geräte bekannt, bei welchen der momentane physiologische Zustand des Patienten dadurch bestimmt wird, daß auf das, über eine Atemgasleitung dem Patienten zugeführte Atemgas definierte Druckimpulse aufgebracht werden, wobei beispielsweise auf Grundlage einer hiermit einhergehenden Impedanzmessung Schlüsse auf den momentanen Obstruktionsgrad gezogen werden können.

Aus WO 97/28838 ist ein System zur Erzeugung eines kontinuierlichen, positiven Atemwegsdrucks bekannt, bei welchem der zeitliche Verlauf des Atemgasstromes analysiert wird und Druckpegel des dem Patienten zugeführten Atemgases nach Maßgabe des Analyseergebnisses angepasst wird. Die Analyse des des Atemgasstromes erfolgt unter Rückgriffnahme auf eine Korrelationsbetrachtung. Bei dieser Korrelationsbetrachtung wird der zeitliche verlauf des Atemgasflusses mit einer Sinus-Funktion verglichen das Vergleichsergebnis gibt Aufschluss über die Eignung des momentanen Atemgasdruckes und ermöglicht insoweit eine ggf. erforderliche Anhebung oder Absenkung desselben.

Hinsichtlich der bislang zur Anwendung gelangten Druck-Regelungskonzepte zur selbsttätigen, patientenbezogenen Anpassung des Beatmungsdruckes besteht das Problem, daß die hierdurch veranlaßten Änderungen des Beatmungsdruckes von den betroffenen Patienten nur beschränkt akzeptiert werden. Zudem besteht das Problem, daß die bekannten Auto-CPAP-Systeme teilweise erst auf erhebliche Atmungsstörungen reagieren.

Der Erfindung liegt die Aufgabe zugrunde, eine Vorrichtung zur Erfassung der Atmungstätigkeit sowie zur Vorgabe physikalischer Parameter bei der Zufuhr eines Atemgases zu einer Person zu schaffen, die eine präzisere Beurteilung des physiologischen Zustandes des Patienten ermöglicht.

Diese Aufgabe wird erfindungsgemäß gelöst eine Vorrichtung gemäß Patentanspruch 1.

Dadurch wird es auf vorteilhafte Weise möglich, eine extrem scharfe Klassifizierung der Atmungstätigkeit der atmenden Person vorzunehmen und hierauf basierend eine auf den physiologischen Zustand des Patienten präzise abgestimmte Einstellung des Beatmungsdruckes physiologisch gut verträgliche Weise vorzunehmen, ohne hierbei das natürliche Schlafverhalten zu stören. Die auf der präzisen Klassifizierung bzw. Detektion der Atmungstätigkeit basierende Drucksteuerung führt zu einer deutlich verbesserten Therapieakzeptanz und ermöglicht eine vorausschauende Anpassung des Beatmungsdruckes, durch welche mit hoher Wahrscheinlichkeit dem Eintritt etwaiger Atemwegsobstruktionen vorgebeugt werden kann.

Auf Grundlage des erfindungsgemäßen Beurteilungskonzeptes wird es auf vorteilhafte Weise möglich, sicherzustellen, daß der durch ein entsprechendes CPAP-Gerät eingestellte Atemgasdruck mit hoher Zuverlässigkeit und ohne besonderen Diagnoseaufwand patientenspezifisch festgelegt werden kann. Durch das erfindungsgemäße Beurteilungskonzept wird es zudem möglich, von einer aktiven Änderung des Beatmungsdruckes, wie sie bislang zur Überwachung des physiologischen Zustands des Patienten erforderlich war, abzusehen und den physiologischen Zustand des Patienten mit hoher Zuverlässigkeit ohne willkürlich hervorgerufene Druckexperimente zu ermitteln.

Erfindungsgemäß ist die Länge der ersten Zeitspanne zur Ermittlung der Referenzbeziehung derart bemessen, daß diese sich über wenigstens zwei Atemzyklen erstreckt. Es ist möglich, die Generierung der Referenzbeziehung durch ein Kriterienfeld zu definieren. Dieses Kriterienfeld enthält vorzugsweise mehrere Einträge, durch welche festgelegt ist, wie die Referenzbeziehung aus den erfaßten ersten und zweiten Signalen gebildet wird. Beispielsweise ist es möglich, für bestimmte Eigenschaften der Referenzbeziehung die über einen längeren Zeitraum erfaßten ersten und zweiten Signale auszuwerten, wogegen für andere Eigenschaften der Referenzbeziehung kürzere Beobachtungszeiträume gewählt sind.

Gemäß einer besonders bevorzugten Ausführungsform der Erfindung ist wenigstens eine Filtereinrichtung vorgesehen, zum Filtern des ersten und/oder zweiten Signales auf Grundlage eines vorbestimmten Frequenzbereiches. Hierdurch wird es möglich, bestimmte erfassungsbedingte Rauscheinflüsse weitgehend auszuschalten.

Gemäß einer weiteren besonders bevorzugten Ausführungsform der Erfindung umfaßt die Signalverarbeitungseinrichtung wenigstens eine Glättungseinrichtung zur Glättung der Referenzbeziehung unter Anwendung vorgegebener Glättungskriterien. Diese Glättungskriterien können gemäß einer besonders bevorzugten Ausführungsform der Erfindung adaptiv abgestimmt werden. Es ist auch möglich, für bestimmte Atmungszustände vorbestimmte Glättungskriterien auszuwählen, oder die Glättungskriterien auf den momentan erfaßten Atmungszustand abzustimmen.

Vorzugsweise werden auch die Parameter der Filtereinrichtung adaptiv abgestimmt. Das Adaptionsverhalten kann vorzugsweise durch Eingabe entsprechender Parameter festgelegt werden.

Die Signalverarbeitungseinrichtung umfasst vorzugsweise eine Zähleinrichtung zum Zählen der Erfüllung vorbestimmter Kriterien innerhalb vorbestimmter Zeitspannen. Die Zeitspannen können ggf. auf den momentanen Atmungszustand variabel abgestimmt werden.

Die Erfassung der hinsichtlich des Atemgasdruckes indikativen Parameter kann beispielsweise über einen Drucksensor erfolgen, der in ein entsprechendes CPAP-Gerät integriert ist und beispielsweise über eine Meßschlauchleitung den statischen Druck im Bereich einer am Patienten applizierten Atemmaske erfaßt. Die hinsichtlich des Atemgasstromes indikativen zweiten Signale können beispielsweise über eine Meßblendenanordnung ermittelt werden, die in einem Atemgasversorgungsweg eingesetzt ist.

Durch die erfindungsgemäß vorgeschlagene Vorrichtung, bzw. auf Grundlage des durch diese Vorrichtung apparativ verwirklichten Analyseverfahrens wird eine robuste Detektion der einzelnen Atemzüge der atmenden Person möglich. In vorteilhafterweise erfolgt der Übergang aus der Inspirationsphase in die Expirationsphase über ein ausgeprägte Flanke, auf deren Grundlage eine sichere Erkennung einzelner Atemzüge ermöglicht wird. In vorteilhafter Weise kann die erste zeitliche Ableitung der Atemgasflußkurve geschätzt werden. Die lokalen Extrema der geschätzten ersten Ableitung entsprechen der maximalen Steigung des Atemflusses beim Übergang zwischen Inspiration und Expiration. Von der Exspiration aus wird der Anfang der Inspiration gesucht, indem eine Suche der vorhergehenden Extrema aus der geschätzten zweiten Ableitung erfolgt. Weitere vorteilhafte Ausgestattungen der Erfindung sind Gegenstand der Unteransprüche.

Die Länge der ersten Zeitspanne ist vorzugsweise derart bemessen, daß sich diese über wenigstens zwei Atemzyklen erstreckt. Vorzugsweise ist eine zweite Einrichtung vorgesehen ist zur Bereitstellung eines hinsichtlich des dynamischen und/oder statischen Atemgasdruckes indikativen zweiten Signales. In vorteilhafter Weise ist wenigstens eine Filtereinrichtung vorgesehen, zur Filterung oder Dämpfung der ersten und/oder zweiten Signale.

Die Signalverarbeitungseinrichtung umfasst vorzugsweise eine Glättungseinrichtung, zur Glättung der Referenzbeziehung unter Anwendung ausgewählter Glättungskriterien. Die Glättungskriterien werden vorzugsweise adaptiv verändert. Die Signalverarbeitungseinrichtung umfaßt vorzugsweise eine Glättungseinrichtung zur Glättung oder Dämpfung der Referenzbeziehung.

Wenigstens eine der genannten Glättungseinrichtungen ist vorzugsweise derart ausgebildet, daß diese eine Glättung auf Grundlage statistischer Ansätze vornimmt. Die Signalverarbeitungseinrichtung umfaßt vorzugsweise eine Schwellwertbetrachtungseinrichtung, zur Auswertung der Korrelationsbeziehung hinsichtlich Schwellwertkriterien, insbesondere Nulldurchgänge. Die Signalverarbeitungseinrichtung umfast in vorteilhafter Weise eine Zähleinrichtung, zum Zählen der Erfüllung vorbestimmter Kriterien innerhalb einer vorbestimmten Zeitspanne. Die Filter- und/oder Glättungsparameter werden vorzugsweise adaptiv angepaßt.

Weitere Einzelheiten und Merkmale ergeben sich aus der nachfolgenden Beschreibung in Verbindung mit der Zeichnung. Es zeigen:
- Fig. 1:: Oben: Datenausschnitt aus der Flußkurve eines Patienten in NREM 2.
Mitte: Die hohe vertikale Linie markiert das Ende der Inspiration, die niedrige vertikale Linie markiert den Beginn der Inspiration.
Unten: Erste Ableitung der Flußkurve anhand der das Ende und der Anfang der Inspiration detektiert werden können;
- Fig. 2:: Oben: Datenausschnitt aus der Flußkurve eines Patienten im NREM 2.
Mitte: Der letzte Atemzyklus aus der oberen Datenreihe wurde als Referenzbeziehung für das Atemflußmuster ausgewählt.
Unten: Korrelation zwischen dem Datenstück oben (Referenz-beziehung) und dem Atemflußmuster in der Mitte.
- **Fig. 3:**: Oben: Datenausschnitt aus der Flußkurve eines Patienten im
NREM 2.
Unten: Mittlere Abweichung der Korrelationsmaxima von 1.
- Fig. 4:: Oben: Datenausschnitt aus der Flußkurve eines Patienten im REM.
Unten: Mittlere Abweichung der Korrelationsmaxima von 1.
- **Fig. 5:**: Oben: Datenausschnitt aus der Flußkurve eines Patienten.
Mitte: Zugehörige CPAP-Druckkurve.
Unten: Varianz des CPAP-Signals pro Atemzug.

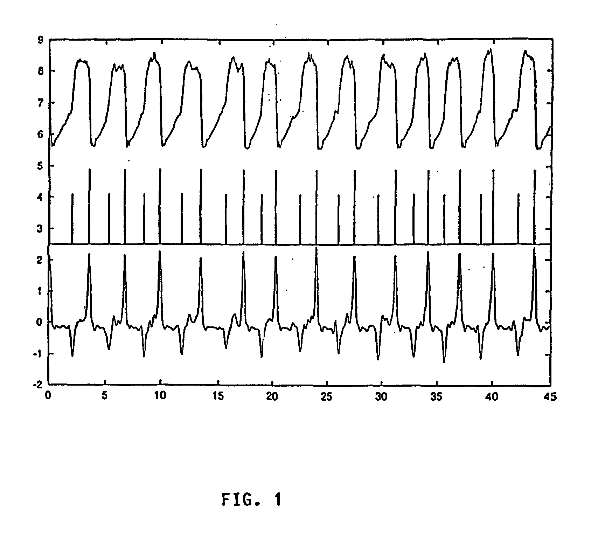

In Fig. 1 sind oben 50 Sekunden der Flußkurve eines Patienten im Schlafstadium NREM 2 dargestellt. Die untere Kurve zeigt die geschätzte erste Ableitung der Flußkurve. Zwischen den beiden Kurven sind die hierbei automatisch detektierten Zeitpunkte durch vertikale Linien markiert.

Zur Unterscheidung zwischen stabiler und nichtstabiler Atmung wird ein Maß für die Ähnlichkeit mehrerer aufeinanderfolgender Atemzyklen betrachtet. Als Maß für die Ähnlichkeit eignet sich hierfür die Höhe einer Kreuzkorrelationsfunktion des aktuellen Atemzyklus mit den vorhergehenden Atemzyklen. Der in Fig.1 dargestellte obere Graph zeigt hierbei eine Atemgas-Flußkurve eines Patienten im NREM 2-Schlafstadium. Die hohe vertikale Linie des mittleren Graphens markiert das Ende der Inspiration, die niedrige vertikale Linie markiert den Beginn der Inspiration. Die erste Ableitung der Flußkurve anhand der das Ende und der Anfang der Inspiration detektiert werden können ist als unterer Graph dargestellt. Aufgrund der unterschiedlichen Extremwerte der ersten Ableitung der Atemgasflusskurve kann zuverlässig zwischen den einzelnen Atemphasen unterschieden werden.

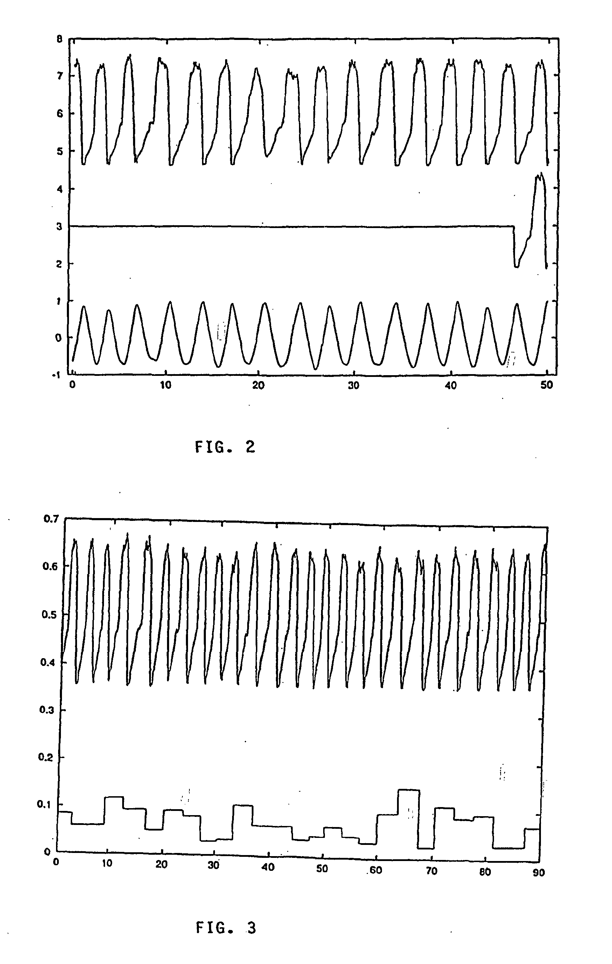

In Fig. 2 ist oben beispielhaft ein 50-Sekunden-Ausschnitt aus einer Atemflußkurve eines Patienten im NREM 2 dargestellt. In der Mitte ist ein ausgewählter Atemzug abgebildet. In der unteren Kurve ist die Korrelation zwischen der Datenreihe oben und dem einzelnen Atemmuster gezeigt. Die Korrelationskurve hat Werte zwischen 1 und -1, wobei die Korrelation gleich 1 wird, wenn die beiden Atemzüge genau aufeinander passen und gleich -1 werden, wenn die Kurven negativ miteinander korreliert sind, d.h., wenn eine Spitze im Atemmuster genau mit einem Tal im betrachteten Datenstück übereinstimmt.

Anhand der Korrelationskurve ist erkennbar, ob 1. die Atmung regelmäßig ist, und ob, 2. Atemzüge ganz fehlen. Wenn die aufeinanderfolgenden Atemmuster ähnlich sind, dann hat die Korrelationskurve einen periodischen Verlauf mit lokalen Maxima nahe bei 1 und lokalen Minima nahe bei -1.

In der in Fig. 2 dargestellten Korreiationskurve wird hierbei an jedem lokalen Maximum die Differenz zu 1 berechnet und über alle so erhaltenen Werte gemittelt. Dieser zwischen 0 und 1 liegendende Mittelwert kann als Maß dafür verwendet werden, wie gut das Atemmuster mit den vergangenen Atemzyklen übereinstimmt.

In Fig. 3 ist oben die Atemflußkurve eines Patienten im NREM 2-Schlaf zu sehen. In der Datenreihe unten ist die mittlere Abweichung der Korrelationsmaxima von 1 dargestellt.

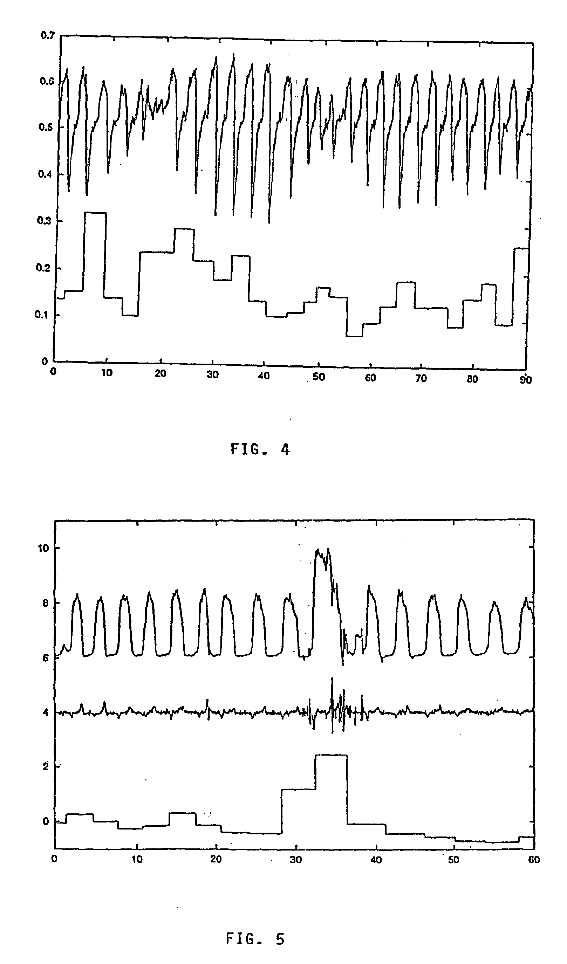

Fig. 4 entspricht im Grunde Fig. 3, jedoch stammt die Flußkurve hier aus dem REM-Schlafstadium. Der Vergleich der mittleren Korrelationsmaxima nach Fig. 3 und Fig. 4 zeigt, daß die mittlere Differenz der Korrelationsmaxima zu 1 im REM-Schlafstadium deutlich größer ist.

Die nachfolgende Tabelle zeigt, welche Gruppe von Atemzuständen sich mit dem vorangehend beschriebenen Ähnlichkeitsmaß unterscheiden lassen.

| **Stabile Atmung** | **Nichtstabile Atmung** |
|---|---|
| | |
| Normalatmung in Ruhe | Irreguläre Atmung im REM |
| Atmung mit Schnarchen | Obstruktive Apnoe |
| Mundatmung | Wachatmung |
| Periodische Atmung mit Flußlimitation | Cheyne Stoke'sche Atmung |
| Gedämpftes Atemflußsignal | |
| | |

### Detektion von Schnarchen

Die Detektion von Schnarchen kann gemäß einer besonders bevorzugten Ausführungsform der Erfindung auf Grundlage der Varianz des CPAP-Druckes in einem Atemzug ermittelt werden. In Abbildung 5 ist oben ein Ausschnitt aus einem Atemflußsignal dargestellt, darunter ist der zugehörige CPAP-Druck abgebildet. In der darunterliegenden Kurve ist die Varianz des CPAP-Druckes pro Atemdruck zu sehen. Die Varianz nimmt deutlich zu, wenn das CPAP-Signal durch Schnarchen des Patienten verändert wird.

### Weitere Unterscheidungsparameter

Auf Grundlage der durch das erfindungsgemäße Konzept zuverlässig detektierten Zeitpunkte für den Anfang und das Ende der Inspiration, können weitere wichtige Merkmale für die Unterscheidung von Atemzuständen gewonnen werden. Besonders vorteilhaft auswertbare Angaben sind hierbei die Inspirationszeit, die Expirationszeit, der maximale Fluß während der Inspiration, der maximale Fluß während der Expiration, das Inspirationsvolumen und das Expirationsvolumen.

### Mundatmung

Artefakte durch Mundatmung können in besonders vorteilhafter Weise zuverlässig erfaßt werden, da in diesem Falle eine negative Korrelation existiert. Obstruktive Apnoen können dadurch erfaßt werden, daß bestimmte Korrelationspeaks deutlich abgeschwächt sind, oder im Regelfall vollständig fehlen.

### Flußlimittierte Atmung

Sofern die Inspiration flußlimittiert ist, kann auf Grundlage des der Erfindung zugrundeliegenden Konzeptes die flußlimittierte Atmung anhand des Inspirationsvolumens oder auch anhand der relativen Änderung des maximalen Inspirationsflusses erkannt werden. Wenn man den ungefähren Anfangs- und Endzeitpunkt der Inspiration kennt, kann man den Zeitpunkt der maximalen Inspiration bestimmen. Wenn dieser Zeitpunkt in der ersten Hälfte der Inspiration liegt, kann mit hoher statistischer Wahrscheinlichkeit auf eine flußlimittierte Inspiration geschlossen werden und eine entsprechende Korrektur des Beatmungsdruckes veranlaßt werden.

### Cheyne Stoke'sche Atmung

Charakteristisch für die Cheyne Stoke'sche Atmung ist der periodische Verlauf der Atmung, der sich in einem periodischen Verlauf des Inspirationsvolumens wiederspiegelt und sich hiermit von den anderen nichtstabilen Atemmustern unterscheiden läßt.

### Kontrolle der detektierten Atemzüge

Die Korrelationskurve kann in besonders vorteilhafter Weise zur Kontrolle der Detektion der Zeitpunkte des Inspirationsbeginns und -endes verwendet werden, da ein lokales Maxima in der Korrelationskurve ein mit hoher statistischer Sicherheit aussagefähiges Merkmal für einen Atemzug darstellt.

Auf Grundlage des der erfindungsgemäßen Vorrichtung zugrundeliegenden Analysekonzeptes wird es möglich, mit statistisch hoher Sicherheit einzelne Atemzüge zu erkennen und weitreichende Rückschlüsse auf den momentanen Zustand des Patienten zu ziehen. Durch die so gewonnenen Informationen wird es möglich, den Therapiedruck vorausschauend und unter vergleichsweise geringen Änderungsgradienten auf die physiologischen Bedürfnisse des Patienten abzustimmen. Hierdurch wird eine besonders hohe Therapieakzeptanz erreicht.

## Patentansprüche

1. Vorrichtung zur Erfassung der Atmungstätigkeit einer Person mit wenigstens einer ersten Einrichtung zur Bereitstellung eines hinsichtlich eines Atemgasstromes v indikativen ersten Signals, und
wenigstens einer Signalverarbeitungseinrichtung zur Verarbeitung des ersten Signals;
wobei die Signalverarbeitungseinrichtung derart ausgebildet ist, daß diese eine Korrelationsbeziehung zwischen einer Referenzbeziehung und dem ersten Signal ermittelt und
auf Grundlage einer Betrachtung wenigstens der Korrelationsbeziehung ein für die Atmungstätigkeit oder den physiologischen Zustand der atmenden Person, indikatives Ausgangssignal erzeugt und die Atemgasdrucksteuerung hierauf abstimmt, **dadurch gekennzeichnet, dass** die Signalverarbeitungseinrichtung jene Referenzbeziehung auf Grundlage des über eine erste Zeitspanne erfassten ersten Signals ermittelt, und dass die Länge der ersten Zeitspanne derart bemessen ist, dass sich diese über wenigstens zwei Atemzyklen erstreckt.

2. Vorrichtung nach Anspruch 1, **dadurch gekennzeichnet, dass** eine zweite Einrichtung vorgesehen ist zur Bereitstellung eines hinsichtlich des dynamischen und/oder statischen Atemgasdruckes indikativen zweiten Signales.

3. Vorrichtung nach Anspruch 1 oder 2, **dadurch gekennzeichnet, dass** die Signalverarbeitungseinrichtung derart konfiguriert ist, dass die Generierung der Referenzbeziehung durch ein Kriterienfeld erfolgt wobei dieses Kriterienfeld mehrere Einträge enthält, durch welche festgelegt ist, wie die Referenzbeziehung aus den erfassten ersten und zweiten Signalen gebildet wird.

4. Vorrichtung nach Anspruch 1, **dadurch gekennzeichnet, dass** die Signalverarbeitungseinrichtung derart konfiguriert ist, dass für bestimmte Eigenschaften der Referenzbeziehung die erfassten ersten und zweiten Signale über einen längeren Zeitraum ausgewertet werden, wogegen für andere Eigenschaften der Referenzbeziehung kürzere Beobachtungszeiträunie gewählt sind.

5. Vorrichtung nach wenigstens einem der Ansprüche 1 oder 2, **dadurch gekennzeichnet, dass** wenigstens eine Filtereinrichtung vorgesehen ist, zur Filterung oder Dämpfung der ersten und/oder zweiten Signale.

6. Vorrichtung nach wenigstens einem der Ansprüche 1 bis 5, **dadurch gekennzeichnet, dass** die Signalverarbeitungseinrichtung eine Glättungseinrichtung umfaßt, zur Glättung der Referenzbeziehung unter Anwendung ausgewählter Glättungskriterien.

7. Vorrichtung nach Anspruch 6, **dadurch gekennzeichnet, dass** die Signalverarbeitungseinrichtung derart konfiguriert ist, dass die Glättungskriterien adaptiv verändert werden.

8. Vorrichtung nach wenigstens einem der Ansprüche 6 oder 7, sofern rückbezogen auf Anspruch 5, **dadurch gekennzeichnet, dass** die Signalverarbeitungseinrichtung derart konfiguriert ist, dass Filter parameter adaptiv angepaßt werden.

9. Vorrichtung nach wenigstens einem der Ansprüche 6 bis 8, **dadurch gekennzeichnet, dass** die Signalverarbeitungseinrichtung derart konfiguriert ist, dass Glättungsparameter adaptiv angepaßt werden.

## Claims

1. A device for detecting the respiratory activity of a person comprising at least one first means for providing a first signal being indicative of a respiratory gas flow v and
at least one signal processing means for processing the first signal;
wherein the signal processing means is configured such that it determines a correlation relation between a reference relation and the first signal and
on the basis of a consideration of at least the correlation relation, generates an output signal being indicative of the respiratory activity or the physiologic condition of the breathing person and adjusts the respiratory gas pressure control thereto, **characterized in that** the signal processing means determines said reference relation on the basis of the first signal detected over the first time period and that the length of the first time period is calculated such that it extends over at least two breathing cycles.

2. The device of claim 1, **characterized in that** a second means is provided for providing a second signal being indicative of the dynamic and/or static respiratory gas pressure(s).

3. The device of claim 1 or 2, **characterized in that** the signal processing means is configured such that the reference relation is generated by a criteria field, wherein this criteria field comprises a plurality of records which define how the reference relation is formed on the basis of the detected first and second signals.

4. The device of claim 1, **characterized in that** the signal processing means is configured such that for specific properties of the reference relation, the detected first and second signals are evaluated over a longer time period while for other properties of the reference relation shorter monitoring time periods are selected.

5. The device according to at least one of claim 1 or 2, **characterized in that** at least one filter means is provided for filtering or dampening the first and/or second signal(s).

6. The device according to at least one of claims 1 to 5, **characterized in that** the signal processing means comprises a smoothing means for smoothing the reference relation by applying selected smoothing criteria.

7. The device according to claim 6, **characterized in that** the signal processing means is configured such that the smoothing criteria are changed adaptively.

8. The device according to at least one of claim 6 or 7, if back-referenced to claim 5, **characterized in that** the signal processing means is configured such that filter parameters are adjusted adaptively.

9. The device according to at least one of claims 6 to 8, **characterized in that** the signal processing means is configured such that smoothing parameters are adjusted adaptively.

## Revendications

1. Dispositif de détection de l'activité respiratoire d'une personne avec au moins une première installation pour émettre un premier signal indiquant un flux de gaz respiratoire v, et
au moins une installation de traitement de signaux pour traiter le premier signal ; moyennant quoi l'installation de traitement de signal est conçue de telle sorte qu'elle établit une relation de corrélation entre une relation de référence et le premier signal et génère un signal de sortie indiquant l'activité respiratoire ou l'état physiologique de la personne qui respire en tenant compte au moins de la relation de corrélation et ajuste la commande de pression de gaz respiratoire en fonction dudit signal de sortie, **caractérisé en ce que** l'installation de traitement de signaux détermine ladite relation de référence en se basant sur le premier signal détecté pendant une première période, et **en ce que** la durée de la première période est définie de façon à ce qu'elle s'étende sur au moins deux cycles respiratoires.

2. Dispositif selon la revendication 1, **caractérisé en ce qu'**il est prévu une seconde installation pour émettre un second signal indiquant la pression dynamique et/ou statique des gaz respiratoires.

3. Dispositif selon la revendication 1 ou 2, **caractérisé en ce que** l'installation de traitement de signaux est configurée de telle sorte que la relation de référence est généré par le biais d'un champ de critères, ce champ de critères comprenant plusieurs entrées permettant de déterminer comment la relation de référence est formée à partir des premier et second signaux détectés.

4. Dispositif selon la revendication 1, **caractérisé en ce que** l'installation de traitement de signaux est configurée de telle sorte que les premier et second signaux détectés sont exploités pour certaines caractéristiques de la relation de référence pendant une longue durée, tandis que pour d'autres caractéristiques de la relation de référence on choisit des durées d'observation plus courtes.

5. Dispositif selon au moins l'une des revendications 1 ou 2, **caractérisé en ce qu'**il est prévu au moins une installation de filtrage pour filtrer ou atténuer les premier et/ou second signaux.

6. Dispositif selon au moins l'une des revendications 1 à 5, **caractérisé en ce que** l'installation de traitement de signaux comprend une installation de lissage pour lisser la relation de référence à l'aide des critères de lissage choisis.

7. Dispositif selon la revendication 6, **caractérisé en ce que** l'installation de traitement de signaux est configurée de telle sorte que les critères de lissage sont modifiés de manière adaptive.

8. Dispositif selon au moins l'une des revendications 6 ou 7 lorsqu'elles se rapportent à la revendication 5, **caractérisé en ce que** l'installation de traitement de signaux est configurée de telle sorte que les paramètres de filtrage sont modifiés de manière adaptive.

9. Dispositif selon au moins l'une des revendications 6 à 8, **caractérisé en ce que** l'installation de traitement de signaux est configurée de telle sorte que les paramètres de lissage sont modifiés de manière adaptive.
